# FASCICULE DE BREVET EUROPEEN

(11) **EP 2 086 891 B1**
(45) Date de publication et mention de la délivrance du brevet: **23.05.2018**
(21) Numéro de dépôt: 07858690.6
(22) Date de dépôt: 13.11.2007
(51) Int. Cl.: C02F 3/28, C12M 1/107

(54) **PROCÉDÉ ET INSTALLATION DE TRAITEMENT ANAÉROBIE DE MATIÈRES À CONCENTRATION DE MATIÈRE SÈCHE ÉLEVÉE**
VERFAHREN UND INSTALLATION ZUR ANAEROBEN BEHANDLUNG VON MATERIAL MIT HOHER FESTSTOFFKONZENTRATION
PROCESS AND INSTALLATION FOR ANAEROBIC TREATMENT OF MATERIAL HAVING A HIGH SOLIDS CONCENTRATION

(30) Priorité: 13.11.2006 FR 0654872; 05.01.2007 FR 0752530
(43) Date de publication de la demande: 12.08.2009
(73) Titulaire: Valorga International, 34935 Montpellier Cedex 09 (FR)
(72) Inventeur: LOTTI, Jean-Pierre, 34130 Valergues (FR); FRUTEAU DE LACLOS, Hélène, 34830 Jacou (FR); SAINT-JOLY, Claude, 34160 Sussargues (FR)
(74) Mandataire: Rhein, Alain
(86) Numéro de dépôt international: PCT/FR2007/052332
(87) Numéro de publication internationale: WO 2008/059167

(56) Documents cités:
- WO-A-2005/077841
- FR-A2- 2 551 457

## Description

L'invention a trait à un procédé et une installation de dégradation anaérobie de déchets contenant des matières organiques solides, sous forme d'une pâte à concentration de matière sèche élevée, dans tous les cas supérieure à 15%, et notamment comprise entre 25% et 30%.

La présente invention concerne plus particulièrement le domaine de la dégradation anaérobie de déchets constitués de matières organiques solides hétérogènes pouvant contenir des particules indésirables, notamment lourdes et non organiques, susceptibles de se sédimenter dans une cuve de fermentation, comme par exemple des cailloux, du verre ou des composés métalliques.

Les déchets organiques solides dont la dégradation est visée par la présente invention sont préalablement préparés sous la forme d'une pâte de concentration en matière sèche élevée, ladite pâte pouvant être fibreuse, mais dans tous les cas compacte.

Dans le domaine de la dégradation anaérobie d'effluents à faible teneur en matières sèches, donc plus liquides que pâteux, il est connu des procédés de dégradation anaérobie utilisant des cuves adaptées.

Le document FR 2 510 605 présente par exemple un procédé et une installation pour la dégradation en milieu anaérobie humide de produits, sous-produits et déchets organiques, comprenant un réacteur ayant une cuve cylindrique de fermentation, divisée verticalement en deux parties par une cloison centrale. La première partie est reliée par un siphon à un puits d'alimentation et la deuxième partie est reliée par un siphon à un puits d'évacuation des matières. L'alimentation et l'évacuation des produits sont réalisées par poussée pneumatique.

Le document FR 2 530 659 reprend cette même structure et se propose de la perfectionner en imposant un sens de circulation tourmenté aux effluents à l'intérieur de la cuve, tout en prévoyant une insufflation de biogaz par jets brefs et successifs par des conduits débouchant dans le fond de la cuve.

Selon un mode de réalisation de cuve décrit dans ces documents les puits d'alimentation et d'évacuation sont situés à proximité l'un de l'autre, la cloison précitée étant disposée verticalement entre les deux orifices débouchant desdits puits dans le réacteur. Cette cloison a une largeur inférieure à la largeur de la cuve et une hauteur inférieure à la hauteur de la cuve, le fond de la cuve présentant une double pente ayant sensiblement la forme d'une ellipse.

Selon un autre mode de réalisation de cuve encore décrit dans ces documents, les puits d'alimentation et d'évacuation précités sont sensiblement diamétralement opposés, la cloison verticale séparant sensiblement diamétralement la cuve de fermentation, avec une hauteur inférieure à la hauteur de la cuve et laissant des passages communiquant entre les deux compartiments en parties supérieure et inférieure afin de favoriser un mouvement ascendant des matières en première partie et un mouvement descendant dans la seconde partie, le fond de la cuve ayant une pente unique.

De manière encore plus spécifique, le document FR 2 551 457 propose de subdiviser l'enceinte en une pluralité de secteurs par l'injection intermittente de biogaz prélevé d'un réservoir de stockage adapté, dans chacun desdits secteurs sous une pression et une période de temps prédéterminés. Le biogaz est réintroduit dans chaque secteur successivement, c'est-à-dire de manière décalée dans le temps, de manière à obtenir une rotation de l'injection de biogaz dans l'enceinte, d'un secteur à l'autre.

Enfin, le document FR 2 577 940 propose de supprimer les puits d'alimentation et d'évacuation des matières afin d'abaisser les coûts de construction. Dans ce cas on réalise l'introduction directe des produits à dégrader dans l'enceinte, de préférence vers le fond de ladite enceinte, et la sortie des produits dégradés se fait par gravité. La poussée mécanique est réalisée par une pompe à matière épaisse, de préférence à piston ou à

L'un des inconvénients des solutions antérieurement connues réside dans la complexité des digesteurs qui en résultent. En particulier ces digesteurs sont de fabrication coûteuse, liée
aux contraintes de cloisonnement interne et aux conceptions spécifiques des moyens d'adduction et d'évacuation d'effluents.

En effet, les cloisonnements réalisés doivent posséder une résistance mécanique importante du fait des pressions exercées par les effluents en mouvement. Il en résulte des coûts de fabrication importants.

De fait, les contraintes de fabrication pesant sur les digesteurs de l'état de la technique sont d'autant plus importantes que la taille de la cuve du digesteur est élevée.

Il résulte encore de cet exposé de l'état de la technique qu'un des problèmes non parfaitement solutionné par les procédés et dispositifs de digestion existants est le contrôle d'une circulation homogène des matières à digérer entre les voies d'alimentation et d'évacuation.

Jusqu'à présent la circulation des matières sous forme d'effluents a été réalisée au travers d'un compartimentage et d'une gestion tourmentée du flux d'effluents à l'intérieur de ladite cuve.

La présente invention se propose de répondre à ces inconvénients de l'état de la technique au travers d'un procédé et d'une installation finalement très simple.

A cet effet l'invention concerne un procédé de traitement anaérobie de déchets contenant des matières organiques solides sous forme d'une pâte compacte à concentration de matière sèche élevée, c'est-à-dire supérieure à 15%, dans un digesteur sous forme d'une cuve cylindrique fermée dépourvue de tout moyen de cloisonnement, ainsi que de tout équipement mécanique interne mais pourvue de moyens d'adduction de matières à traiter et de moyens d'évacuation des matières digérées ainsi que de moyens d'homogénéisation verticale constitués par des rampes d'injection de fluide gazeux sous forme d'injecteurs d'un fluide gazeux dans le fond de la cuve transversalement à la direction sensiblement horizontale du flux de matières dans la cuve, caractérisé en ce que
- les moyens d'adduction de matières à traiter et les moyens d'évacuation de matières digérées sont constitués par des orifices répartis à différentes hauteurs de la paroi de la cuve selon un arc de cercle sur la section circulaire de ladite cuve et diamétralement opposés les uns par rapport aux autres,
- des moyens d'homogénéisation verticale garantissant l'homogénéité des matières par secteurs verticaux dans la cuve, et grâce à une alimentation par poussée on impose aux matières dans la cuve une circulation unidirectionnelle forcée uniforme sur toute la section de cette dernière, et selon une composante sensiblement horizontale, entre lesdits moyens d'adduction et lesdits moyens d'évacuation.

Avantageusement, l'espace interne de ladite cuve est découpé en secteurs verticaux par la disposition des moyens d'homogénéisation et l'on homogénéise les secteurs verticaux par les moyens d'homogénéisation de manière intermittente et successive.

On peut encore réaliser une extraction préférentielle des matières sédimentées en bas de la cuve et/ou une recirculation des matières entre les moyens d'évacuation et les moyens d'adduction.

Selon une autre caractéristique, cette recirculation peut être faite pendant de brèves périodes.

L'invention concerne encore une installation pour la mise en oeuvre du procédé, comportant un digesteur sous forme d'une cuve cylindrique fermée dépourvue de tout cloisonnement, ainsi que de tout équipement mécanique interne mais pourvue de moyens d'adduction de matières à traiter et de moyens d'évacuation des matières digérées ainsi que de moyens d'homogénéisation verticale constitués de rampes d'injection de fluide gazeux sous forme d'injecteurs d'un fluide gazeux s'étendant dans le fond de la cuve transversalement à la direction sensiblement horizontale du flux de matière dans la cuve, caractérisée en ce que :
les moyens d'adduction de matières à traiter et moyens d'évacuation des matières digérées sont constitués par des orifices respectivement d'alimentation et d'évacuation qui sont répartis à différentes hauteurs sur la paroi de la cuve et sont disposés selon un arc de cercle sur la section circulaire de la cuve et que les moyens d'adduction de matières à traiter sont disposés au niveau de la cuve de manière diamétralement opposée aux moyens d'évacuation des matières digérées, de manière à imposer aux matières dans la cuve, par une alimentation par poussée, une circulation unidirectionnelle forcée uniforme dans un sens horizontal sur toute la section de la cuve.

Selon une autre caractéristique, les moyens d'adduction de matières et les moyens d'évacuation des matières digérées sont disposés selon un arc de cercle sur la section circulaire de la cuve. Ils peuvent encore être disposés à différentes hauteurs de la cuve.

Avantageusement, les injecteurs de fluide gazeux sont disposés sur des rampes parallèles entre elles et perpendiculaires au sens de progression des matières dans la cuve.

L'installation peut encore comprendre un dispositif d'évacuation des matières digérées, en particulier un dispositif permettant leur déshydratation complémentaire.

Selon une autre caractéristique, l'installation peur comprendre en plus un circuit de recirculation des matières entre les moyens d'évacuation et les moyens d'adduction.

L'invention permet ainsi de traiter dans des conditions favorables, par digestion, des produits organiques solides hétérogènes, par exemple des déchets ménagers et assimilés (déchets urbains, industriels, agricoles etc...), avec des teneurs en matière sèche élevée, par exemple de l'ordre de 25% à 30%.

Grâce à la présente invention la construction des ouvrages sera simplifiée et le coût de fabrication radicalement diminué. Par ailleurs une gamme plus large de matériaux sera utilisable pour ladite construction.

D'autres buts et avantages de la présente invention apparaîtront à la description qui va suivre. La compréhension de cette description sera facilitée en se référant aux dessins ci-joints, dans lesquels :
- la figure 1 est une représentation schématisée et en élévation d'une cuve selon une première variante de réalisation,
- la figure 2 est une représentation schématisée de la section d'une cuve formant digesteur selon l'installation de l'invention,
- la figure 3 est une représentation schématisée des rampes d'injection de fluide gazeux s'étendant dans le fond d'une cuve,
- la figure 4 est une représentation schématisée en élévation d'une cuve selon une deuxième variante de réalisation,
- la figure 5 est une vue d'une disposition particulière d'un injecteur de fluide gazeux au niveau d'une bouche d'évacuation de matières située en partie basse d'une cuve,
- la figure 6 est une représentation schématisée en élévation d'une cuve pourvue de moyens d'accélération des matières dans la cuve.
Tel que visible dans les figures des dessins ci-joints, la présente invention concerne un procédé et une installation de traitement anaérobie de déchets à concentration de matière sèche élevée. A ce propos, on notera que l'invention concerne spécifiquement le traitement de tels déchets contenant des matières organiques solides, sous forme d'une pâte à concentration de matière sèche élevée, dans tous les cas supérieure à 15%, et notamment comprise entre 25% et 30%, et donc composés de matières à faible teneur en eau.

Les déchets organiques solides dont la dégradation est visée par la présente invention sont préalablement préparés, notamment par l'ajout d'un liquide, par exemple des eaux issues de la déshydratation des matières digérées, sous la forme d'une pâte de concentration en matière sèche élevée, ladite pâte pouvant être fibreuse, mais dans tous les cas compacte.

Ces déchets peuvent en outre, sans que cela soit sytématique, contenir des particules lourdes non organiques susceptibles de se sédimenter dans une cuve de fermentation.

Sa concentration en matière sèche élevée confère à la pâte destinée à être traitée dans le cadre de l'invention une viscosité telle que les phénomènes de décantation, même s'ils existent, sont limités.

Comme les matières utilisées se présentent sous forme d'une pâte, les termes de « matières » et de « pâte » seront donc indifféremment utilisés à titre générique pour désigner toutes ces matières susceptibles d'être traitées au travers de la présente invention.

Selon le procédé de l'invention, la dégradation anaérobie de matières organiques solides à forte teneur en matière sèche, dans tous les cas supérieure à 15%, et notamment comprise entre 25% et 30%, est réalisée dans une installation comportant une cuve 1 de fermentation cylindrique verticale, dépourvue de tout cloisonnement, ainsi que de tout équipement mécanique interne.

Au niveau de cette cuve 1, il est prévu des moyens d'adduction 2 des matières à traiter et des moyens d'évacuation 3 des matières digérées, les moyens 2 et 3 étant constitués par des orifices respectivement d'alimentation 2 et d'évacuation 3.

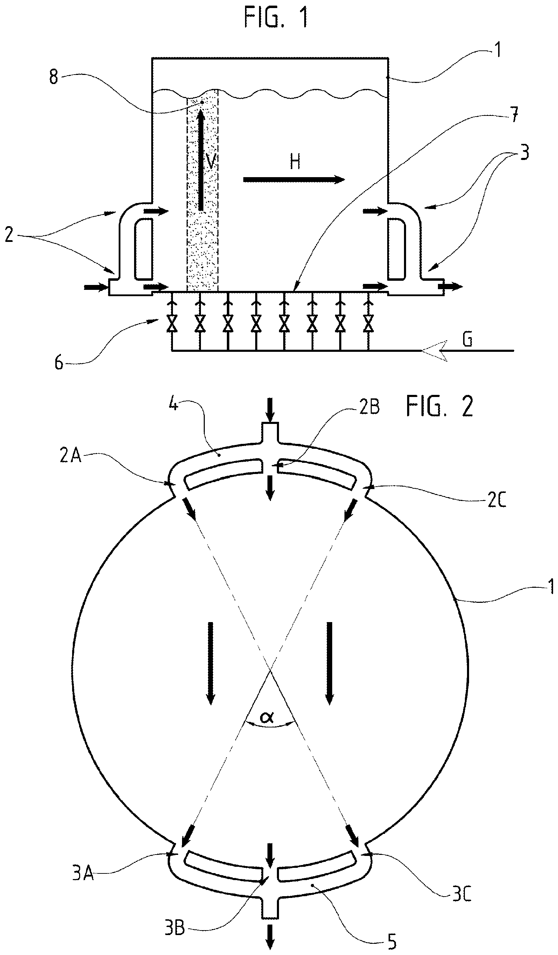

Tels qu'illustrés sur les figures 1 et 2, les orifices d'alimentation 2 et d'évacuation 3 sont disposés de manière à maintenir un cheminement forcé unidirectionnel des matières dans un plan sensiblement horizontal, et sur toute la section de la cuve 2. En effet, le ou les orifices 2 d'alimentation en matières à traiter sont placés au niveau de la paroi de cette cuve 2, de forme circulaire, de façon sensiblement diamétralement opposée à l'orifice ou aux orifices d'évacuation 3 des matières fermentées. Une circulation forcée unidirectionnelle est ainsi imposée aux matières en fermentation dans un sens horizontal grâce à une alimentation par poussée, obtenue de préférence par l'utilisation d'une pompe, par exemple à piston ou à vis.

La figure 2 illustre une réalisation préférentielle d'une telle cuve 1, où les orifices d'alimentation 2A, 2B, 2C ainsi que les orifices de sortie 3A, 3B, 3C sont répartis sur un arc de cercle dont l'angle inscrit a, dans tous les cas inférieur à 180°, est déterminé de telle façon que les matières qui entrent dans la cuve soient réparties sur une surface importante de la section de la cuve. Le sens de circulation des matières est matérialisé par des flèches.
Avantageusement, les orifices 2A, 2B, 2C d'un côté, et 3A, 3B, 3C de l'autre sont reliés par des canalisations uniques 4 et 5, afin d'assurer un débit d'alimentation et/ou de sortie des matières au travers de ces orifices uniforme, et obtenir des vitesses de progression des matières similaires sur toute la section de la cuve.

D'autre part, comme illustré sur la figure 1, les orifices d'alimentation 2A, 2B, 2C et/ou de sortie 3A, 3B, 3C sont répartis à différentes hauteurs sur la paroi de la cuve.

La circulation est en outre favorisée par des moyens d'homogénéisation 6 sous forme de buses d'injection 6 de fluide gazeux sous pression au niveau du fond 7 de la cuve 1.

On comprend que l'injection de gaz sous pression induit une homogénéisation par secteurs verticaux 8 dans la cuve, en imprimant un mouvement vertical ascendant aux matières sur toute la hauteur de la cuve 1. Les secteurs verticaux 8 considérés sont définis par l'emplacement des buses d'injections et prennent avantageusement la forme de tranches parallèles.

Les moyens d'homogénéisation 6 sont un élément primordial dans le fonctionnement du procédé et de l'installation selon la présente invention. Sur la figure 1 sont représentées les deux composantes du mouvement des matières en fermentation : la progression horizontale H et le mouvement vertical V.

La progression horizontale H est obtenue sous l'action de la poussée de l'alimentation des matières à traiter.

Le mouvement vertical V, favorisant le fait que les matières en cours de traitement ne sédimentent pas, est produit par l'injection de fluide, notamment de gaz, sous pression G à la base de la cuve.

Avantageusement, la pression d'injection du fluide à la base de la cuve est supérieure ou égale à deux fois la pression statique dans la cuve. Par exemple, pour une hauteur de matières de 20 mètres à l'intérieur de la cuve, la pression d'injection est supérieure ou égale à 4 bars.

C'est justement en limitant la sédimentation dans un secteur et dans la cuve en général, et en diminuant le risque de voir apparaître de vitesses différentielles de circulation entre les matières, notamment à l'intérieur d'un même secteur 8 et dans la cuve en général, que les moyens d'homogénéisation verticale contribuent à l'avancée homogène des matières injectées dans la cuve, cette avancée correspondant globalement à celle des secteurs verticaux 8.
La division virtuelle de la cuve 1 en plusieurs secteurs 8 est réalisée par le biais de la répartition des buses d'injection 6 dans le fond de la cuve 1 de fermentation. Chaque secteur 8 est alimenté individuellement en gaz sous pression. Les gaz sont injectés successivement dans chaque secteur 8 et de manière décalée dans le temps.

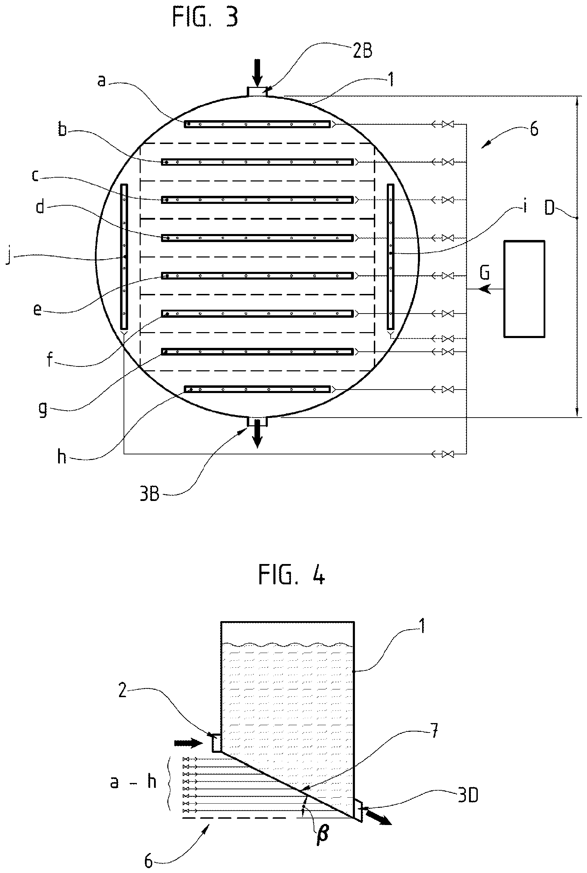

La figure 3 illustre plus particulièrement la disposition de ces secteurs 8. Ceux-ci sont agencés de manière à ce que la base de la cuve soit découpée virtuellement par des rampes, numérotées de a à h, parallèles entre elles et perpendiculaires au sens de progression des matières dans la cuve 1, en somme perpendiculaires au plan D reliant les orifices médians d'alimentation 2B et d'évacuation 3B. Ainsi l'injection dans chaque secteur 8 successivement se fait depuis la rampe a du côté des orifices d'alimentation 2A, 2B, 2C, vers la rampe h du côté opposé correspondant aux orifices d'évacuation 3A, 3B, 3C, favorisant un mouvement dans le sens de la circulation unidirectionnelle et horizontale forcée des matières.
De manière avantageuse, des rampes i et j perpendiculaires aux précédentes et localisées sur les bords de la cuve 1 complètent ces moyens d'homogénéisation 6. Ceci peut se révéler particulièrement favorable pour les cuves de très grand volume, en limitant les volumes morts dans la cuve 1 de digestion, ce qui est essentiel pour le bon fonctionnement du procédé et l'installation pour la mise en oeuvre du procédé.
De manière générale l'absence de cloisonnement constitue dans l'invention un avantage pour la circulation des matières. D'un point de vue économique, cette absence se traduit également par une diminution du coût de fabrication de la cuve 1.

Selon une deuxième variante de réalisation d'une cuve 1 illustrée en figure 4, le fond 7 de la cuve 1 présente une pente, ladite pente étant orientée de telle façon que les matières en fermentation, et notamment, si elles sont présentes, les particules lourdes éventuellement sédimentés sur ce fond de la cuve, cheminent par gravité depuis l'orifice ou les orifices d'alimentation 2A, 2B, 2C vers l'orifice ou les orifices de d'évacuation 3A, 3B, 3C. L'angle de la pente β est ajusté à la nature et à la granulométrie de la pâte à traiter, afin que le cheminement soit progressif et compatible avec la transformation des matières organiques sous l'action de la fermentation.

Avantageusement, un 3D au moins des orifices d'évacuation est situé dans la partie basse de la paroi de la cuve 1 de telle façon que si des particules lourdes sont accumulées au point bas du fond 7 de la cuve 1, elles sortent de l'enceinte par gravité.

Selon cette deuxième variante de réalisation, les buses 6 au travers desquelles est injecté le fluide sous pression s'étendent horizontalement dans le fond 7 en pente de cette cuve 1 et de telle façon que le fluide soit dirigé dans la même direction que le cheminement des matières. Les avantages de cette disposition sont énoncés ci-après. Premièrement cette disposition permet de créer dans le périmètre proche de la buse une poussée pneumatique dans le sens de la circulation des matières, qui favorise le cheminement transversal desdites matières et notamment des particules lourdes éventuellement sédimentées sur le fond 7 de la cuve 1. Deuxièmement on évite ainsi la pénétration par gravité de ces particules lourdes dans l'orifice des buses 6.

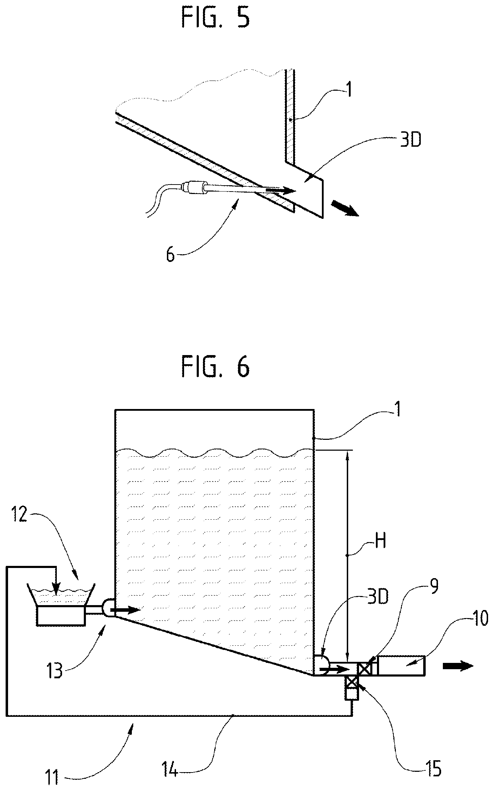

On disposera avantageusement des buses d'injection 6 de fluide sous pression en regard de l'orifice ou des orifices 3D d'évacuation situés en point bas de la cuve 1, comme illustré sur la figure 5. De cette façon on autorise, au travers de ces buses 6, une action mécanique ou pneumatique, destinée à désobstruer le cas échéant tout ou partie de l'orifice ou des orifices 3D, dans le cadre de la maintenance de l'ouvrage, sans intervention directe sur lesdits orifices susceptible de représenter une perturbation majeure dans le fonctionnement du présent dispositif et procédé.

Selon une autre caractéristique, la présente invention tire parti de la géométrie de la cuve 1 pour réaliser d'une part une extraction et d'autre part un recyclage gravitaires des matières fermentées.

Ainsi, le procédé selon l'invention permet encore une extraction préférentielle des inertes lourds éventuellement sédimentés au point bas de la cuve de fermentation.

On rappelle à ce propos que le procédé et dispositif selon l'invention sont destinés à traiter des matières constitués de déchets organiques solides hétérogènes pouvant contenir des particules indésirables susceptibles de se sédimenter dans une cuve de fermentation, comme par exemple des cailloux, du verre ou des composés métalliques.

Comme illustré sur la figure 6, au moins un orifice de sortie 3D est relié par l'intermédiaire d'une vanne 9 à un dispositif 10 permettant l'évacuation des matières digérées, avantageusement conçu sous forme d'un dispositif 10 permettant leur déshydratation complémentaire.

On rappelle à ce propos que les eaux issues de cette déshydratation peuvent être utilisées pour la préparation des déchets organiques solides dont la dégradation est visée par la présente invention, pour la préparation de la pâte avec laquelle est alimentée l'installation.

Dans la configuration selon la présente invention, les particules lourdes éventuellement sédimentées dans le point bas de la cuve 1 sont, après ouverture de la vanne 9, entraînées par gravité dans le flux de matières, et ceci préférentiellement dans une première phase de soutirage. Le flux correspondant à cette première phase est donc dirigé vers le dispositif 10 permettant l'évacuation des matières digérées.

D'un autre côté, afin d'ensemencer les produits avant leur entrée dans la cuve 1, il est prévu un circuit de recirculation 11 d'une partie des matières digérées vers les orifices d'alimentation 2.

A ce propos, on rappelle que selon l'esprit de l'invention, celle-ci recherche une circulation uniforme des matières dans la cuve. Il est ensuite possible d'envisager le contrôle de la vitesse de circulation des matières dans la cuve.

Le procédé et dispositif selon l'invention se rapportant à un procédé et dispositif de digestion en continu, il en résulte que pour une quantité donnée de matières entrant dans le système on soutire une quantité équivalente de matières digérées.

Ainsi, une autre caractéristique avantageuse de la présente invention est, lors du recyclage des matières fermentées à fort débit, de conférer audites matières une vitesse de circulation élevée de telle façon d'entraîner les particules lourdes éventuellement sédimentées dans les tuyauteries.

L'installation pour la mise en oeuvre du procédé selon l'invention comporte, pour le circuit de recirculation 11, un raccordement extérieur à la cuve 1, intervenant entre les moyens d'évacuation 3 et les moyens 2 d'alimentation en matières à traiter.

Selon une caractéristique de l'invention illustrée sur la figure 6, au moins un orifice de sortie 3D est relié à un dispositif de stockage 12, travaillant à pression atmosphérique, constitué par une trémie d'alimentation située sur une pompe d'introduction 13, via une tuyauterie 14 équipée d'au moins une vanne 15 à contrôle automatique. L'ouverture de cette vanne 15 permet la communication directe entre la cuve de fermentation et le stockage tampon constitué par le dispositif 12.

Ainsi la pression statique générée par la hauteur H des matières en fermentation dans la cuve 1 est transmise directement aux matières à l'intérieur de la tuyauterie 14.

En choisissant de manière adéquate le diamètre des tuyauteries, on obtient un débit de matières durant la période d'ouverture de ladite vanne 15 très élevé, beaucoup plus élevé que le débit qui pourrait être assuré seulement avec une pompe du même type que celle servant à l'alimentation des matières. Ce débit élevé génère une vitesse de circulation des matières élevée, de telle façon que les particules lourdes éventuellement sédimentées sont entraînées dans le flux de matières. On évite ainsi une obstruction des tuyauteries.

Une séquence adaptée d'ouvertures et de fermetures successives de la vanne permet d'obtenir des débits élevés sur de courtes périodes, et donc ponctuellement une vitesse de circulation des matières dans les tuyauteries élevée, tout en garantissant un débit moyen résultant choisi.

Afin de parfaire le contrôle du débit, une pompe peut néanmoins avantageusement compléter ce dispositif.

## Revendications

1. Procédé de traitement anaérobie de déchets contenant des matières organiques solides sous forme d'une pâte compacte à concentration de matière sèche élevée, c'est-à-dire supérieure à 15%, dans un digesteur sous forme d'une cuve (1) cylindrique fermée dépourvue de tout cloisonnement, ainsi que de tout équipement mécanique interne mais pourvue de moyens d'adduction (2) de matières à traiter et de moyens d'évacuation (3) des matières digérées ainsi que de moyens d'homogénéisation (6) verticale constitués par des rampes (a, b, c, d, e, f, g, h) d'injection de fluide gazeux sous forme d'injecteurs d'un fluide gazeux dans le fond (7) de la cuve (1) transversalement à la direction sensiblement horizontale du flux de matières dans la cuve, **caractérisé en ce que**
- les moyens d'adduction (2) des matières à traiter et les moyens d'évacuation des matières digérées sont constitués par des orifices répartis à différentes hauteurs sur la paroi de la cuve (1) selon un arc de cercle sur la section circulaire de ladite cuve (1) et diamétralement opposés les uns par rapport aux autres,
- des moyens d'homogénéisation (6) verticale garantissant l'homogénéité des matières par secteurs verticaux (8) dans la cuve, et grâce à une alimentation par poussée on impose aux matières dans la cuve (1) une circulation unidirectionnelle forcée uniforme sur toute la section de cette dernière, et selon une composante sensiblement horizontale, entre lesdits moyens d'adduction (2) et lesdits moyens d'évacuation (3).

2. Procédé selon la revendication 1, l'espace interne de ladite cuve (1) étant découpé en secteurs verticaux (8) par la disposition des moyens d'homogénéisation (6), **caractérisé en ce que** l'on homogénéise les secteurs verticaux (8) par les moyens d'homogénéisation (6) de manière intermittente et successive.

3. selon l'une des revendications 1 ou 2, **caractérisé en ce que** l'on réalise une extraction préférentielle des matières sédimentées en bas de la cuve (1).

4. Procédé selon l'une des revendications 1 à 3, **caractérisé en ce que** l'on réalise une recirculation des matières entre les moyens d'évacuation (3) et les moyens d'adduction (2).

5. Procédé selon la revendication 4, **caractérisé en ce que** l'on réalise la recirculation des matières entre les moyens d'évacuation (3) et les moyens d'adduction (2) pendant de brèves périodes.

6. Installation pour la mise en oeuvre du procédé selon les revendications 1 à 5, comportant un digesteur sous forme d'une cuve (1) cylindrique fermée dépourvue de tout cloisonnement, ainsi que de tout équipement mécanique interne mais pourvue de moyens d'adduction (2) de matières à traiter et de moyens d' évacuation (3) des matières digérées, ainsi que de moyens d'homogénéisation (6) verticale constitués de rampes (a, b, c, d, e, f, g, h) d'injection de fluide gazeux sous forme d'injecteurs d'un fluide gazeux s'étendant dans le fond (7) de la cuve (1) transversalement à la direction sensiblement horizontale du flux de matières dans la cuve (1), **caractérisée en ce :**
- **que** les moyens d'adduction (2) de matières à traiter et les moyens d'évacuation (3) des matières digérées sont constitués par des orifices respectivement d'alimentation et d'évacuation qui sont répartis à différentes hauteurs sur la paroi de la cuve (1) et sont disposés selon un arc de cercle sur la section circulaire de la cuve (1) et que les moyens d'adduction (2) de matières à traiter sont disposés au niveau de la cuve (1) de manière diamétralement opposée aux moyens d' évacuation (3) des matières digérées, de manière à imposer aux matières dans la cuve (1), par une alimentation par poussée, une circulation unidirectionnelle forcée uniforme dans un sens horizontal sur toute la section de la cuve (1).

7. Installation selon la revendication 6, comportant un digesteur sous forme d'une cuve (1) fermée pourvue de moyens d'homogénéisation (6) verticale, **caractérisée en ce que** les injecteurs de fluide gazeux sont disposés sur des rampes (a, b, c, d, e, f, g, h) parallèles entre elles et perpendiculaires au sens de progression des matières dans la cuve (1).

8. Installation selon l'une des revendications 6 et 7, **caractérisée en ce qu'**elle comprend en plus un dispositif (10) d'évacuation des matières digérées, en particulier un dispositif (10) permettant leur déshydratation complémentaire.

9. Installation selon l'une des revendications 6 à 8, **caractérisée en ce qu'**elle comprend en plus un circuit de recirculation (11) des matières entre les moyens d'évacuation (3) et les moyens d'adduction (2).

## Patentansprüche

1. Verfahren zur anaeroben Behandlung von Abfällen, die feste organische Stoffe in Form einer kompakten Paste mit hoher Trockensubstanzkonzentration, d.h. höher als 15%, enthält, in einem Faulbehälter in Form eines geschlossenen zylindrischen Behälters (1) ohne jegliche Raumteilung sowie jegliche innere mechanische Ausrüstung, sondern der mit Mitteln zum Zuführen (2) von zu behandelnden Substanzen und Mitteln zum Abführen (3) der zersetzten Substanzen sowie Mitteln zur vertikalen Homogenisierung (6), die aus Rampen (a, b, c, d, e, f, g, h) zum Einspritzen von gasförmigem Fluid in Form von Einspritzdüsen für gasförmiges Fluid auf dem Boden (7) des Behälters (1) quer zur im wesentlichen horizontalen Richtung des Substanzenflusses im Behälter bestehen, versehen ist, **dadurch gekennzeichnet dass**:
- die Mittel zum Zuführen (2) der zu behandelnden Substanzen und die Mittel zum Abführen der zersetzten Substanzen aus Löchern bestehen, die auf verschiedenen Höhen der Wand des Behälters (1) in einem Kreisbogen auf dem kreisförmigen Querschnitt des besagten Behälters (1) verteilt und diametral gegenüberliegend sind,
- Mittel zur vertikalen Homogenisierung (6) der Substanzen die Homogenität pro vertikale Sektoren (8) in dem Behälter gewährleisten, und durch einem schubweisen Zufuhr den Substanzen in dem Behälter (1) einen gleichmäßigen erzwungen unidirektionalen Umlauf über den gesamten Querschnitt des letzteren und gemäß einer im wesentlichen horizontalen Komponente, zwischen den besagten Zufuhrmitteln (2) und den besagten Abfuhrmitteln (3) auferlegt wird.

2. Verfahren nach Anspruch 1, bei dem der Innenraum des besagten Behälters (1) durch die Anordnung der Homogenisierungsmittel (6) in vertikale Sektoren (8) geschnitten wird, **dadurch gekennzeichnet, dass** die vertikalen Sektoren (8) durch die Homogenisierungsmittel (6) intermittierend und aufeinanderfolgend homogenisiert werden.

3. Verfahren nach einem der Ansprüche 1 oder 2, **dadurch gekennzeichnet, dass** eine bevorzugte Extraktion der abgesetzten Substanzen am Boden des Behälters (1) durchgeführt wird.

4. Verfahren nach einem der Ansprüche 1 bis 3, **dadurch gekennzeichnet, dass** eine Rückführung der Substanzen zwischen den Abfuhnmitteln (3) und den Zufuhrmitteln (2) durchgeführt wird.

5. Verfahren nach Anspruch 4, **dadurch gekennzeichnet, dass** die Rückführung der Substanzen zwischen den Abfuhrmitteln (3) und den Zufuhrmitteln (2) während kurze Zeiträume durchführt wird.

6. Anlage zur Durchführung des Verfahrens nach den Ansprüchen 1 bis 5, umfassend einen Faulbehälter in Form eines geschlossenen zylindrischen Behälters (1) ohne jegliche Trennung sowie jegliche interne mechanische Ausrüstung, sondern versehen mit Mitteln zum Zuführen (2) von zu verarbeitenden Substanzen und Mitteln zum Abführen (3) der zersetzten Substanzen sowie Mitteln zur vertikalen Homogenisierung (6), die aus Einspritzrampen (a, b, c, d, e, f, g, h) für gasförmiges Fluid in Form von Einspritzdüsen für gasförmiges Fluid, die sich am Boden (7) des Behälters (1) quer zur im Wesentlichen horizontalen Richtung des Materialflußes in dem Behälter (1) erstrecken, **dadurch gekennzeichnet, dass**:
- die Mittel zum Zuführen (2) von zu behandelnden Substanzen und die Mittel zum Abführen (3) der zersetzten Substanzen aus Zu- bzw. Abfuhrlöchern bestehen, die auf verschiedenen Höhen an der Wand des Behälters (1) verteilt und in einem Kreisbogen auf dem kreisförmigen Querschnitt des Behälters (1) angeordnet sind, und daß die Mittel zum Zuführen (2) der zu behandelnden Substanzen im Bereich des Behälters (3) den Mitteln zum Abführen (3) der zersetzten Substanzen diametral gegenüberliegend angeordnet sind, sodass sie den Substanzen in dem Behälter (1) durch einen schubweisen Zufuhr einen gleichmäßigen erzwungenen unidirektionalen Umlauf in horizontaler Richtung über den gesamten Querschnitt des Behälters (1) auferlegen.

7. Anlage nach Anspruch 6, umfassend einen Faulbehälter in Form eines geschlossenen Behälters (1), der mit vertikalen Homogenisierungsmitteln (6) versehen ist, **dadurch gekennzeichnet, dass** die Einspritzer für gasförmiges Fluid auf Rampen (a, b, c, d, e, f, g, h) angeordnet sind, die parallel zueinander und senkrecht zur Vorschubrichtung der Substanzen in dem Behälter (1) verlaufen.

8. Anlage nach einem der Ansprüche 6 und 7, **dadurch gekennzeichnet, dass** sie außerdem eine Vorrichtung (10) zum Abführen des zersetzten Substanzen, insbesondere eine Vorrichtung (10), die ihre ergänzende Dehydratierung erlaubt, umfasst.

9. Anlage nach einem der Ansprüche 6 bis 8, **dadurch gekennzeichnet, dass** sie außerdem einen Rückfuhrkreislauf (11) für die Substanzen zwischen den Ablfulirmitteln (3) und den Zufuhrmitteln (2) umfasst.

## Claims

1. Method for anaerobically treating waste containing solid organic materials in the form of a compact paste with a high dry matter concentration, i.e. higher than 15%, in a digester in the form of a closed cylindrical tank (1) without any partitioning means as well as any internal mechanical equipment, but provided with means for supplying (2) materials to be treated and means for discharging (3) the digested materials as well as means for vertical homogenization (6) formed of gaseous fluid injection ramps (a, b, c, d, e, f, g, h) in the form of gaseous fluid injectors on the bottom (7) of the tank (1) arranged transversally to the substantially horizontal direction of the flow of materials in the tank, wherein:
- the means for supplying (2) materials to be treated and the means for discharging digested materials are formed by holes distributed at different heights of the wall of the tank (1) in an arc of a circle on the circular cross-section of said tank (1) and diametrically opposite each other,
- vertical homogenization means (6) guaranteeing the homogeneity of the materials by vertical sectors (8) in the tank, and thanks to a supply by episodes a uniform unidirectional flow is imposed to the materials in the tank (1) over the entire cross-section of the latter, and according to a substantially horizontal component, between said supply means (2) and said discharge means (3).

2. Method according to claim 1, the internal space of said tank (1) being cut into vertical sectors (8) by the arrangement of the homogenization means (6), wherein the vertical sectors (8) are intermittently and successively homogenized by the homogenizing means (6).

3. Method according to one of claims 1 or 2, wherein a preferred extraction of materials settled on the bottom of the tank (1) is performed.

4. Method according to one of claims 1 to 3, wherein a recirculation of the materials between the discharge means (3) and the supply means (2) is performed.

5. Method according to claim 4, **characterized in that** the recirculation of the materials between the discharge means (3) and the supply means (2) is performed for short periods.

6. Plant for implementing the method according to claims 1 to 5, including a digester in the form of a closed cylindrical tank (1) without any partitioning as well as any internal mechanical equipment, but provided with means for supplying (2) materials to be treated and means for discharging (3) the digested materials as well as vertical homogenizing means (6) formed of gaseous fluid injection ramps (a, b, c, d, e, f, g, h) in the form of gaseous fluid injectors extending on the bottom (7) of the tank (1) transversely to the substantially horizontal direction of the material flow in the tank (1), wherein:
- the means for supplying (2) materials to be treated and the means for discharging (3) the digested materials are formed by supply and discharge holes, respectively, which are distributed at different heights of the wall of the tank (1) and are arranged in a circular arc on the circular cross-section of the tank (1) and the means for supplying (2) materials to be treated are arranged at the level of the tank (1) diametrically opposite the means for discharging (3) the digested material, so as to impose to the materials in the tank (1), through a supply by episodes, a uniform unidirectional forced circulation in a horizontal direction over the entire cross-section of the tank (1).

7. Plant according to claim 6, including a digester in the form of a closed tank (1) provided with vertical homogenization means (6), wherein the gaseous fluid injectors are arranged on ramps (a, b, c, d, e, f, g, h) parallel to each other and perpendicular to the direction of progression of the materials in the tank (1).

8. Plant according to one of claims 6 and 7, wherein it comprises in addition a device (10) for discharging the digested materials, in particular a device (10) permitting their complementary dehydration.

9. Plant according to one of claims 6 to 8, wherein it comprises in addition a circuit for recirculating (11) the materials between the discharge means (3) and the supply means (2).
